# EUROPEAN PATENT APPLICATION

(11) **EP 3 252 791 A1**
(43) Date of publication of application: **06.12.2017**
(21) Application number: 16743159.2
(22) Date of filing: 18.01.2016
(51) Int. Cl.: H01G 9/20, C07D 213/74

(54) **DYE-SENSITIZED SOLAR CELL**

(30) Priority: 26.01.2015 JP 2015011901
(71) Applicant: Tanaka Kikinzoku Kogyo K.K., Chiyoda-ku Tokyo 100-6422 (JP)
(72) Inventor: INOUE, Kenichi, Tsukuba-shi Ibaraki 300-4247 (JP); SATO, Hiroki, Tsukuba-shi Ibaraki 300-4247 (JP)
(74) Representative: Weickmann & Weickmann PartmbB
(86) International application number: PCT/JP2016/051297
(87) International publication number: WO 2016/121555

(57) **Abstract**

The present invention relates to a dye-sensitized solar cell, in which there is applied an asymmetric ruthenium complex where two thiocyanate groups and two bipyridine ligands which are different from each other are coordinated with a Ru metal, as a photosensitizing dye, and there is applied an organic compound where two carboxyl groups are coordinated with a hydrocarbon group which has a linear saturated hydrocarbon having 1 or more and 3 or less carbon atoms as a main chain, as a co-adsorbent. The present invention contributes to the improvement of power generation efficiency under a low illumination circumstance, such as an indoor living room, to extend the use of the dye-sensitized solar cell which is excellent in design property. The dye-sensitized solar cell according to the present invention exhibits a high power generation efficiency even under a low illumination circumstance, such as an ordinary living room indoors.

## Description

### TECHNICAL FIELD

The present invention relates to a dye-sensitized solar cell, which is preferably used under a low illumination circumstance indoors.

### BACKGROUND ART

A dye-sensitized solar cell (hereinafter, referred to as DSC) is a solar cell made from an organic material, and generates electric power by utilizing light absorption of an organic dye. The DSC can be made see-through, in comparison with a silicon solar cell (single crystal silicon solar cell, or multi-crystal silicon solar cell, etc.) which has already been widely put to practical use, and is superior in colorful colors and various designs according to its use. In addition, it has advantages that it is easy to be produced, and can be mass-produced inexpensively.

A commonly-used configuration of the DSC includes an electrode, as its center, such as a transparent electrode where an oxide layer (generally made of titanium oxide or the like) which carries a dye material is provided on a substrate, a counter electrode opposite to the electrode, and an electrolyte (generally a iodide solution is used) disposed between the electrode and the counter electrode. The dye carried on the oxide absorbs light to generate electrons and supplies the electrons to the oxide, thus converting the light energy to the electric energy. Further, the electrolyte has a function as a reducing agent that supplies the electrons to the oxidized dye through electron supply.

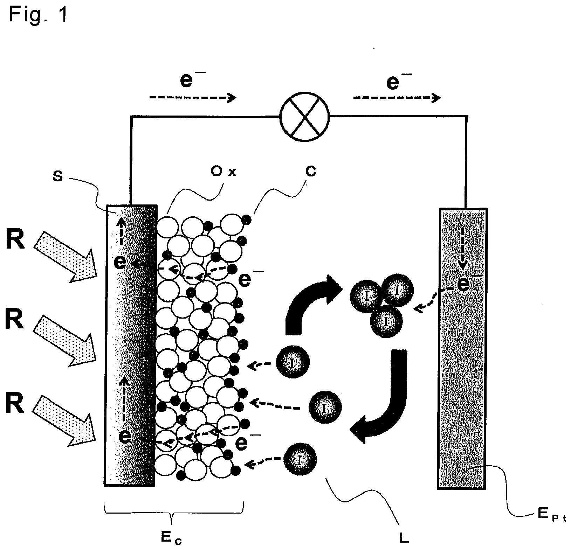

The fundamental power generation principal of the DSC will be explained with reference to Fig. 1. Fig. 1 schematically illustrates the power generation principal of DSC. As shown in Fig. 1, in the electrode, the electrons generated by adsorbing the light by the dye are injected to the oxide, and then flow to the counter electrode through the substrate and an external circuit. On the surface of the counter electrode, the electrons are supplied to the iodine molecule and the like of the electrolyte to be a negative ion, and the iodine ion supplies the electrons to the dye which is oxidized through the light absorption, and then the dye is regenerated and also the iodine ion returns to the initial molecular state. Repeating the above cycle regenerates the dye while inhibiting the decomposition of the dye by oxidation, and thus brings about a state which allows repeated light absorption.

Since the performance (power generating efficiency) of the DSC depends on the properties of the carried dye, various compounds have been developed as such dyes. As the pioneer dye material used at the time of development of the DSC, for example, the following ruthenium complex (N3 dye) was found (PTL 1). Then, Z907 dye which was developed in order to improve the properties of the N3 dye and is the similar compound thereof also has been conventionally known. Further, recently, for such a dye material, there have been reported CYC-based ruthenium complexes which have a high light absorption coefficient (molar light absorption coefficient ε) and a high power generation ability in comparison with the conventional products. Examples of the CYC-based ruthenium complexes include the following CYC-B11, CYC-B1, and the like.

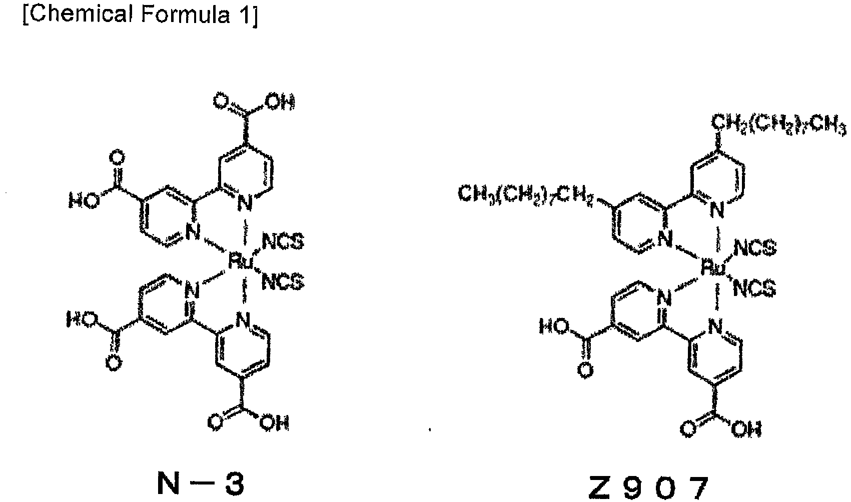

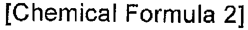

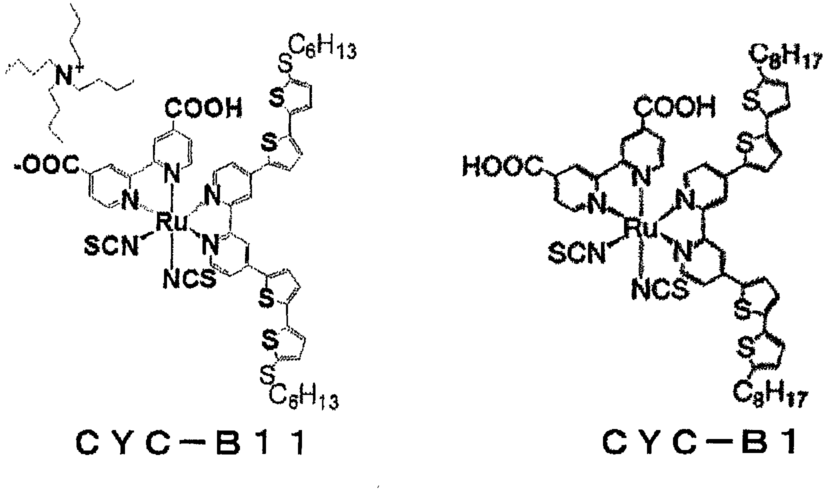

### CITATION LIST

### PATENT LITERATURE

PTL 1: WO 2012/118044 pamphlet

### SUMMARY OF INVENTION

### TECHNICAL PROBLEM

Here, the use of the DSC is desired for not only outdoor but also indoor use. The reason is that, in comparison with the conventional silicon solar cells which mainly use sun light as a light source, the DSC has a relatively high conversion efficiency even when a fluorescent lamp or an LED in the room is used as a light source. In the indoor circumstance, since the light source is weak in irradiation intensity and the wavelength of the light source is limited to a visual light region in comparison with the strong light energy such as sun light, it is very important to improve the properties (power generation efficiency) of the DSC. Further, in order to utilize the above excellent design property of the DSC, it is desired that the power generation efficiency is high not only when used under a high irradiation condition such as pachinko parlors and convenience stors, but also when used under a low irradiation condition such as an ordinary living room, and namely, it is preferable that the high power generation efficiency can be maintained in the wide irradiation range, such as 10 to 2500 lux.

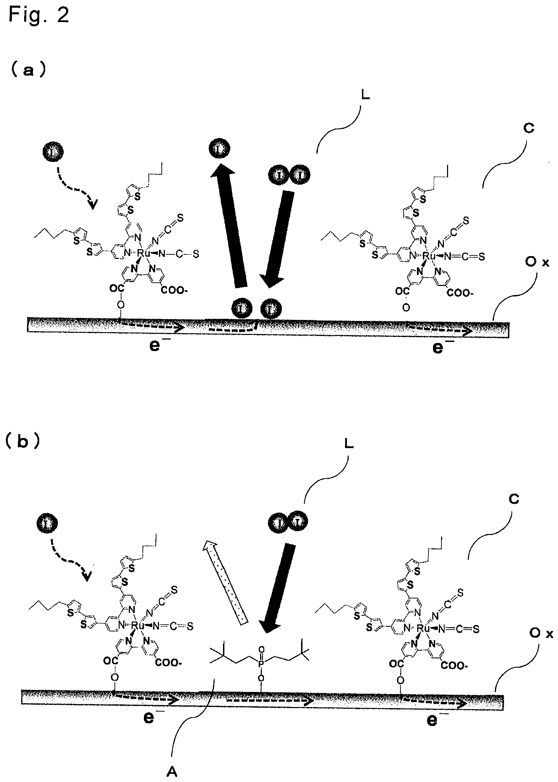

Under such requirements, there has been known, as measures to improve the power generation efficiency, a DSC where an organic compound to inhibit association of the dye molecular (co-adsorbent) is carried on an oxide layer together with the dye as described in PTL 1. This is because it is considered that reduction in power generation efficiency is caused by a phenomenon where an electron which is generated by the light absorption of the dye flows from the oxide layer to the electrolyte (reverse electron transfer reaction), which is reverse to the original purpose that the electron flows to the counter electrode. Here, the reverse electron transfer reaction will be explained by the use of Fig. 2. Fig. 2 schematically shows the carrying state of the dye and the co-adsorbent on the oxide, and the flow of the electron to the electrolyte. In Fig. 2, (a) shows the case of not using the co-adsorbent, and (b) shows the case of using the co-adsorbent. As shown in (a), in the case of not using the co-adsorbent, electron donation to the electrolyte is likely to occur on the surface of the oxide where the surface is exposed because the dye is not carried,. On the other side, in the case of using the co-adsorbent, as in (b), it is considered that the oxide with the exposed surface is decreased by the co-adsorbent carried on the oxide, and thus the electron donation to the electrolyte is decreased.

As explained above, since the reverse electron transfer reaction to the electrolyte is likely to occur when the arrangement of the dye is deviated, and the oxide surface where no dye is carried on is increased, the co-adsorbent has been used in the DSC. Particularly, in the case of using a dye which is asymmetric and bulky, since the association and segregation of the dyes are likely to occur, it is considered that the necessity to add the co-adsorbent is high. However, when the present inventors have confirmed the inhibition effect of the reverse electron transfer reaction by the addition of the co-adsorbent in case of the DSC where the dye which is asymmetric and bulky is used, under a relatively high illumination circumstance of about 1000 lux or more, as in the above hypothesis, it is easy to suppress the reduction in power generation efficiency, but under a relatively low illumination circumstance of about 200 to 500 lux, there has been a case that the reduction in power generation efficiency cannot be suppressed even by the addition of the co-adsorbent.

Accordingly, it is an object of the present invention to provide a DSC which can easily maintain a high power generation efficiency even under a low illumination circumstance such as an ordinary living room in a house, when the dye which is asymmetric and bulky is applied to the DSC.

### SOLUTION TO PROBLEM

In order to solve the above problems, the present inventors have studied as to a co-adsorbent which is also preferable to the case where the dye which is asymmetric and bulky is applied while a co-adsorbent is used in the DSC. To begin with, there has been conventionally and generally used a relatively large co-adsorbent having the same size as the dye. For example, as the known co-adsorbent, there are DINHOP (bis-(3,3-dimethyl-butyl)-phosphinic acid), DPA (decyl phosphonic acid), and the like as shown in the following chemical formulae, and these co-adsorbents have a relatively large molecular size, molecular weight, or number of carbon atoms, and the like. To the contrary, as a result of the present inventors' study, it has been found that when the dye is asymmetric and bulky, instead, a co-adsorbent having a small molecular size, and the like tends to be preferable. This is ascribable to that the bulky dye is particularly easy to be irregular in an arrangement state, thereby there may be generated extremely narrow spaces and wide spaces as the oxide surface on which no dye is carried. It is considered that when the co-adsorbent having a relatively small molecular size, and the like is used, the co-adsorbent can be readily arranged densely even in such irregular spaces on the oxide.

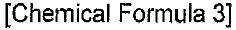

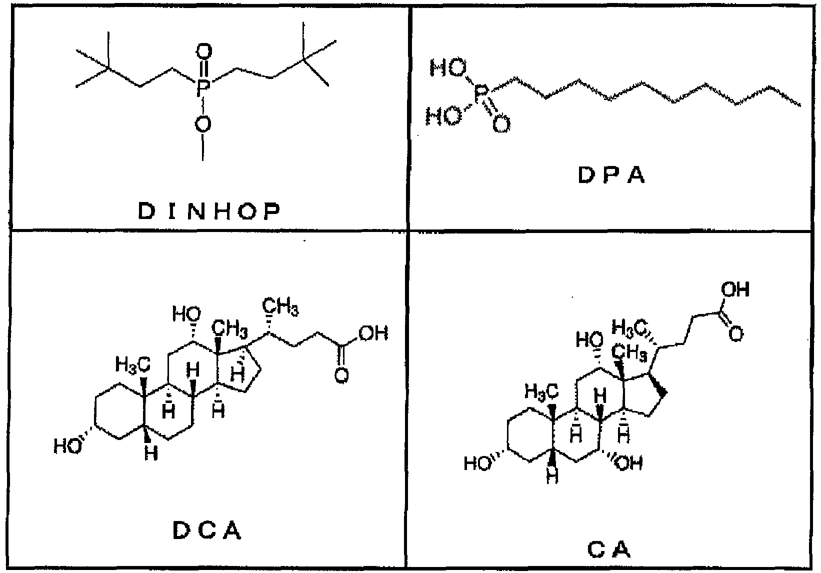

Based on the above study, investigations were made on the DSC to which asymmetric and bulky dye was applied by applying a co-adsorbent having a certain molecular size, a certain molecular weight, or a certain number of carbon atoms or less. There were some instances that the reduction in power generation efficiency cannot be suppressed only by using the co-adsorbent having a low molecular size, and the like. Therefore, with respect to the co-adsorbent, it is necessary to consider other factors other than the size condition. As a result, the present inventors have studied substituents (particularly terminal group) of the co-adsorbent from the viewpoint of degree of bonding of the co-adsorbent to the oxide. From the above, the present inventors have reached the present invention where, in the DSC using the asymmetric and bulky dye, by applying, as the co-adsorbent, an organic compound that both of the size of the molecular and the like and the substituent are optimized, it is possible to obtain the DSC having a high power generation efficiency even under a low illumination circumstance.

Namely, the present invention relates to a dye-sensitized solar cell (DSC) which includes an electrode where an oxide layer which carries a photosensitizing dye and a co-adsorbent is provided on a substrate, a counter electrode opposite to the electrode, and an electrolyte between the electrode and the counter electrode, wherein the photosensitizing dye is an asymmetric ruthenium complex shown in the following general formula (1) where two thiocyanate groups and two bipyridine ligands which are different from each other are coordinated with a Ru metal, and the co-adsorbent is an organic compound shown in the following general formula (2) where two carboxyl groups are coordinated with a hydrocarbon group (Y) which has a linear saturated hydrocarbon having 1 or more and 3 or less carbon atoms as a main chain.

Ruthenium complex represented by the general formula (1) 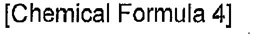 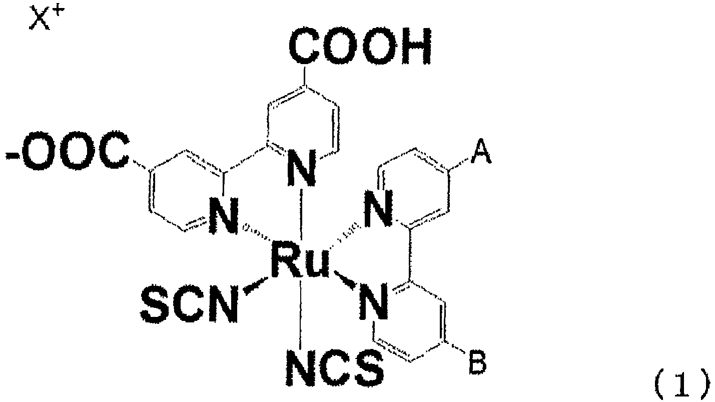 (wherein A and B are a linear hydrocarbon group (having 1 or more and 12 or less carbon atoms), or a substituent which has one or more heterocycles with sulfur or oxygen as a hetero atom. x is a monovalent cation which can be dissociated in a solvent.)

Organic compound represented by the general formula (2) 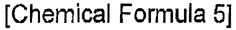 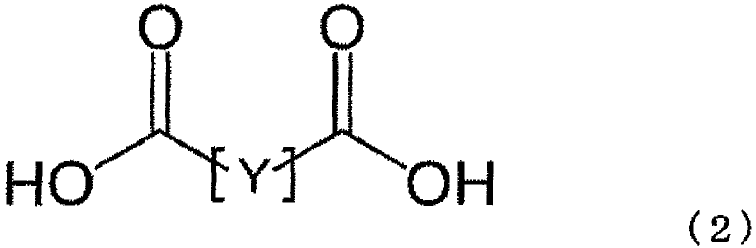 (wherein Y is a hydrocarbon group which is a linear saturated hydrocarbon having 1 or more and 3 or less carbon atoms as a main chain.)

Hereinafter, each configuration of the DSC according to the present invention will be explained in detail.

Firstly, the electrode will be explained among the electrode, the counter electrode and the electrolyte in the DSC of the present invention. The electrode is provided with the oxide layer on the substrate, and the oxide carries the photosensitizing dye and the co-adsorbent. The present invention employs, as the co-adsorbent, the organic compound where the substituents (terminal groups) are two carboxyl groups and the hydrocarbon group sandwiched by these carboxyl groups is a linear saturated hydrocarbon having 1 or more and 3 or less carbon atoms as a main chain.

When the main chain of the hydrocarbon group (Y) has 1 or more and 3 or less carbon atoms, the DSC easily has a high power generation efficiency. It is ascribable to that, even when the carrying state of the photosensitizing dye is deviated, the size of the co-adsorbent is properly small enough to be arranged regularly in the fine area on the surface of the oxide. The number of the carbon atoms of the main chain is preferably 1 or more and 2 or less. When the number of carbon atoms is 4 or more, it is difficult to obtain the effect of suppressing the reverse electron transfer reaction and thus it is difficult to maintain high power generation efficiency.

As mentioned above, the configuration of the co-adsorbent is, as in the general formula (2), the carboxyl groups (-COOH) are coordinated at the both ends of the hydrocarbon group (Y). When the co-adsorbent having two carboxyl groups is applied as described above, the obtained DSC tends to have a high power generation efficiency. This is ascribable to that when a plurality of carboxyl groups bonded to the oxide allows the oxide surface to be more certainly covered by the co-adsorbent.

In the co-adsorbent of the present invention, as mentioned above, as far as the configuration is that the hydrocarbon having a linear main chain which has a certain number of the carbon atoms is sandwiched by two carboxyl groups, the following side chains may be substituted. Namely, the side chain of the hydrocarbon group (Y) preferably has a substituent selected from hydrogen atom, a chain or cyclic hydrocarbon group or hydroxyl group (-OH), particularly preferably has a substituent selected from hydrogen atom, methyl group, benzyl group, and hydroxyl group.

Preferable examples of the co-adsorbent include malic acid, 2-methylmalonic acid, benzylmalonic acid, succinic acid, 2-methylsuccinic acid, glutaric acid, tartaric acid (L-body, D-body, or meso-body). Hereinafter, the chemical structures of the preferable co-adsorbents will be shown.

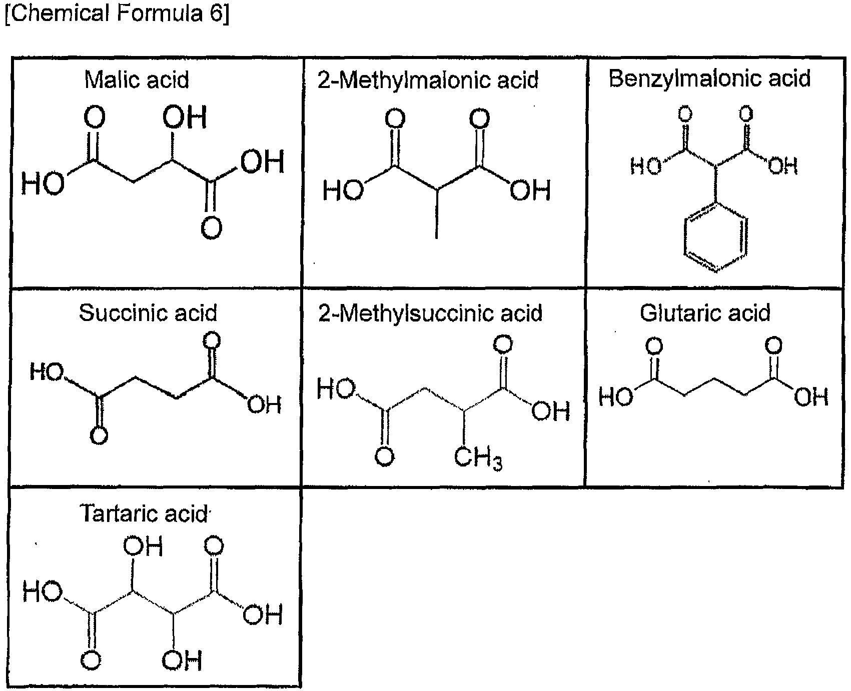

The photosensitizing dye which is carried on the oxide together with the above co-adsorbent is the ruthenium complex represented by the general formula (1). As mentioned above, the present invention, even in the case of using the asymmetric and bulky photosensitizing dye, provides the DSC having a high power generation efficiency by specifying the combination of the photosensitizing dye and the co-adsorbent. The configuration of the photosensitizing dye applied to the present invention is that two thiocyanate groups (-NCS) and two bipyridine ligands are coordinated to the center Ru metal, The two bipyridine ligands are different from each other, and one bipyridine ligand has two carboxylic acid groups, and the other bipyridine ligand has a substituent which is a linear hydrocarbon group, or a substituent which has one or more heterocycles with sulfur or oxygen as a hetero atom. Therefore, the co-adsorbent of the present invention has asymmetric structure since the two different bipyridine ligands are coordinated. 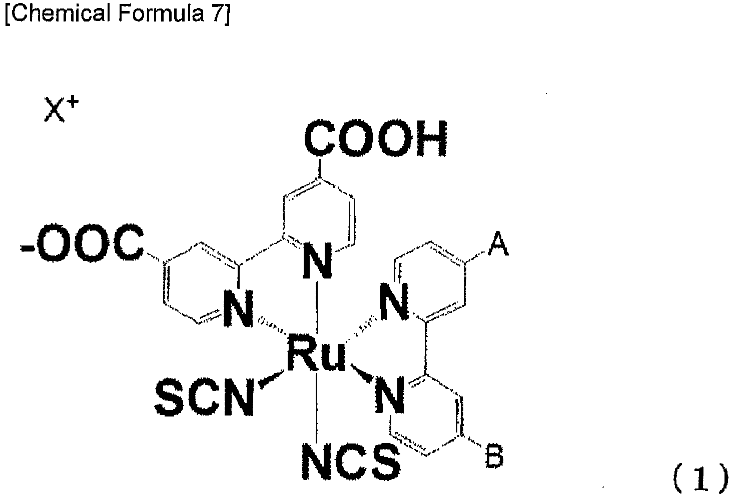 (wherein A and B are a linear hydrocarbon group (having 1 or more and 12 or less carbon atoms), or a substituent which has one or more heterocycles with sulfur or oxygen as a hetero atom. x is a monovalent cation which can be dissociated in a solvent.)

The substituents A and B which are coordinated with the bipyridine group of the photosensitizing dye are a linear hydrocarbon group (having 1 or more and 12 or less carbon atoms), or a substituent which has one or more heterocycles with sulfur or oxygen as a hetero atom. The substituents A and B may be substituents different from each other, but preferably are the same substituents. Preferable examples of the A and/or B have a heterocycle of 5-membered ring or 6-membered ring. Specific examples include thiophene group, furan group, biothiophene group, ethylenedioxythiophene group, and the like, and it is particularly preferable to have thiophene group or biothiophene group. Moreover, the A and/or B is preferably a saturated hydrocarbon group having 1 or more and 12 or less (preferably 6 or more and 9 or less) carbon atoms, or a biothiophene group having a thioether group which has a saturated hydrocarbon group having 1 or more and 9 or less (preferably 6 or more and 8 or less) carbon atoms as a side chain.

The X in the photosensitizing dye is the monovalent cation which can be dissociated in a solvent. The X can form a salt or an acid as a pair of the carboxyl anion (-COO⁻), and corresponds to a so-called counter ion. Examples of the X include a hydrogen ion, an alkali metal ion, an alkyl ammonium ion ((CₙH₂ₙ₋₁)₄N⁺, n being 1 or more and 6 or less), and the like. Preferable examples of the alkali metal ion include lithium ion (Li⁺), sodium ion (Na⁺), and potassium ion (K⁺), and preferable examples of the alkyl ammonium ion include tetra-n-butyl ammonium ion ((CH₃CH₂CH₂CH₂)₄N⁺). Among them, particularly preferable are hydrogen ion, potassium ion, and tetra-n-butyl ammonium ion.

Examples of the particularly preferable photosensitizing dye include CYC-B11 and CYC-B1. Hereinafter, the chemical structures will be shown. Both of the CYC-B11 and CYC-B1 have a biothiophene group as the substituents A and B. The CYC-B1 is a ruthenium complex which has a saturated hydrocarbon group having 8 carbon atoms as the side chain of the biothiophene group, and the X is hydrogen atom. Moreover, the CYC-B11 is a ruthenium complex where a thioether group which has a saturated hydrocarbon group having 6 carbon atoms as a side chain of the biothiophene group is coordinated. The X in the CYC-B11 is a potassium ion or tetra-n-butyl ammonium ion. In the following chemical structures, the dye where the X is potassium is assumed to be K-type, and the dye where the X is tetra-n-butyl ammonium is assumed to be U-type. It is particularly preferable to use the K-type dye as the CYC-B11. According to the study of the present inventors, the K-type dye often deteriorates the power generation efficiency under a low illumination circumstance, however combination with the co-adsorbent according to the present invention tends to inhibit easily power generation efficiency from deteriorating.

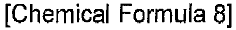

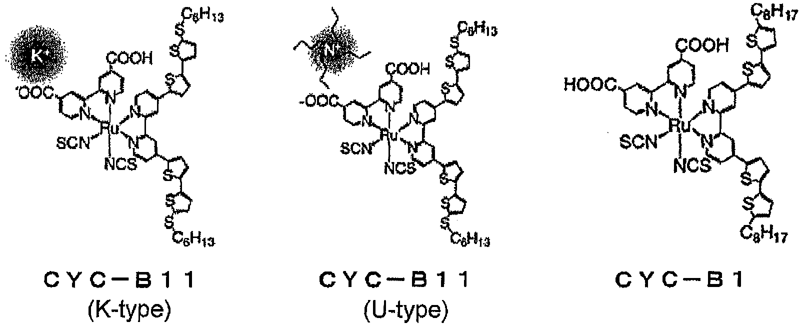

As mentioned above, the present invention is characterized in the combination of the photosensitizing dye and the co-adsorbent, but, when a proportion of each material is within a certain range, it is easy to obtain a DSC having a high power generation efficiency. From such a point of view, it is preferable that a molar ratio of the photosensitizing dye and the co-adsorbent (photosensitizing dye / co-adsorbent) which are carried on the oxide layer is 0.1 or more and 5 or less. When the molar ratio is less than 0.1, there is a tendency that the adsorption of the dye compound which contributes to the power generation is prevented, and when the molar ratio is more than 5, it is difficult to suppress the reverse electron transfer.

In addition, it is allowable to include a cocktail dye on the oxide layer for enlarging the region of corresponding wavelength of the DSC in addition to the aforementioned photosensitizing dye and the co-adsorbent. Examples of the cocktail dye include organic materials such as D35 dye, D131 dye, D149 dye, and D209 dye.

The aforementioned oxide layer which carries the photosensitizing dye and the co-adsorbent is composed of an oxide such as titanium oxide, zirconium oxide, vanadium oxide, manganese oxide, zinc oxide, niobium oxide, molybdenum oxide, indium oxide, tin oxide, tantalum oxide, or tungsten oxide. Particularly preferable is titanium oxide. The oxide layer may be configured by a single layer or may be formed by a multi-layered construction according to the function. When forming the oxide layer by a multi-layered construction, for example, in order to reflect light which cannot be caught by a transparent layer, a scattering layer and a reflecting layer which reflect light in a diffused manner may be provided additionally, while maintaining the transparent layer as a main functional layer where the dye material is carried on and light is converted to an electron. When forming the multi-layered structure, while using the same material, it is possible to characterize the functions by adjusting a particle size, a particle size distribution of the oxide.

In many cases, examples of the substrate which forms the above oxide layer include a glass, and an organic plastic such as PET (polyethylene terephthalate), PEN (polyethylene naphthalate), PES (polyethylene sulfide), PO (polyolefin), or PI (polyimide). This is because the substrate preferably has a light transmittance, since the substrate is a body for supporting the oxide layer and, very often functions as a light receiving surface, in the DSC. In addition, since the organic plastic is flexible, it is possible to produce a DSC having a flexible form. A transparent electrode film such as ITO or FTO may be formed on the surface of the substrate made of such a material. Note that, in many cases, a thickness of the transparent conductive film is 0.1 to 2.0 µm.

For a counter electrode which is provided opposite to the electrode which has the oxide layer on the substrate, platinum, carbon, an electrically conductive polymer film, or the like are used. For an electrolyte which is charged between the electrode and the counter electrode, an electrolytic solution prepared by using iodine, bromine, a cobalt complex or a copper complex may be employed, and an electrolyte of iodine is preferable.

The DSC of the present invention explained above can be produced, for example, by forming the oxide layer on the substrate, and then contacting a solution containing the dye material and the co-adsorbent to the oxide layer to carry the dye and the co-adsorbent on the pores of the oxide layer. The oxide layer is formed preferably, for example, by applying a treating solution which is prepared by dispersing or dissolving the oxide powder in a proper solvent to the substrate, and subjecting the treating solution to drying and sintering to accumulate. Note that, when forming the multiple oxide layers, it is preferable to perform application and sintering of the treating solution multiple times.

The dye and the co-adsorbent may be added to the same solution to be carried on the oxide at a time, or may be added to separate solutions to be carried in a stepwise manner. Moreover, the cocktail dye also may be added to a similar solution to be carried on.

The dye solution may be prepared by dissolving a dye in a solvent such as DMF (N,N-dimethylformamide), GBL (γ-butyrolactone), DMSO (dimethylformamide), DMAc (N,N-dimethylacetoamide), DEF (N,N-diethylformamide), slufolane, N-methylpyrrolidone, dioxane, THF (tetrahydrofurane), dioxolane, dimethyl carbonate, or diethyl carbonate.

### ADVANTAGEOUS EFFECTS OF INVENTION

As explained above, the dye-sensitized solar cell (DSC) of the present invention has a high power generation efficiency even under a low illumination circumstance such as an ordinary living room. Particularly, also in the case that the asymmetric bulky photosensitizing dye is used, it is possible to suppress the reduction in power generation efficiency.

### BRIEF DESCRIPTION OF DRAWINGS

Fig. 1 is a schematic view to explain the basic principal of power generation of the DSC.
Fig. 2 is a schematic view to explain a principal of the reverse electron transfer reaction.

### DESCRIPTION OF EMBODIMENTS

Hereinafter, preferred embodiment of the present invention will be explained.

After formation of an oxide layer on a substrate, a DSC was produced by adsorbing a dye solution which contained various co-adsorbents together with a photosensitizing dye and carrying the dye and co-adsorbents on the oxide layer. Detailed conditions are as follows.
- Substrate
   Glass substrate (size 15 x 25 mm, thickness 1.8 mm)
   FTO film (sheet resistance 15Ω/□)
- Oxide layer (single layer)
   TiO₂ (particle size 15 to 30 nm): 10 µm
- Dye solution
   Dye: CYC-B11 K-type
   Solvent: DMAc
   Co-adsorbent: as in Table 1
   Dye concentration: 0.30 mM%
   Molar ratio: (dye/co-adsorbent) 1/1
   Temperature: 25°C

**[Table 1]**

| No | Co-adsorbent | No | Co-adsorbent |
|---|---|---|---|
| 1 | Malic acid | 6 | Tartaric acid |
| | 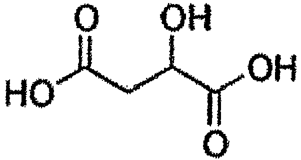 | | 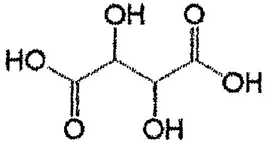 |
| 2 | Benzylmalonic acid. | 7 | Levulinic acid |
| | 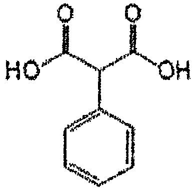 | | 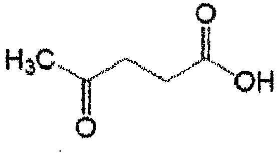 |
| 3 | Succinic acid | 8 | 4-Guanidino butyric acid |
| | 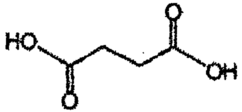 | | 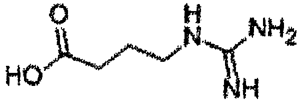 |
| 4 | 2-Methylsuccinic acid | 9 | No co-adsorbent |
| | 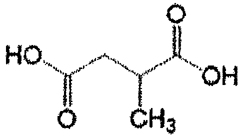 | | |
| 5 | Glutaric acid | | |
| | 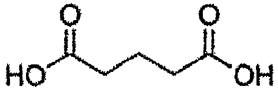 | | |

The DSC was produced by, firstly, applying a treating solution where the above TiO₂ powder was dispersed on a substrate, drying at 120°C, and then sintering at 450°C to form an oxide layer. After dipping the substrate in a dye solution for 60 minutes and taking it out, a platinum plate was pasted as a counter electrode, and a space between the two electrodes was charged with an electrolyte which contained iodine to configure a DSC.

With respect to the produced DSC, the properties of a short-circuit current density (J_{sc}), an open circuit voltage (V_{oc}), a form factor (FF), and a power density (P_{dens}) were evaluated. These measurements were conducted by measuring a J-V property with a solar simulator (YSS-80A) manufactured by YAMASHITA DENSO Co., Ltd., under radiation conditions of a simulated solar light (100 mW/cm²) or an LED light (1000 lux, 200 lux). The results are shown in Table 2.

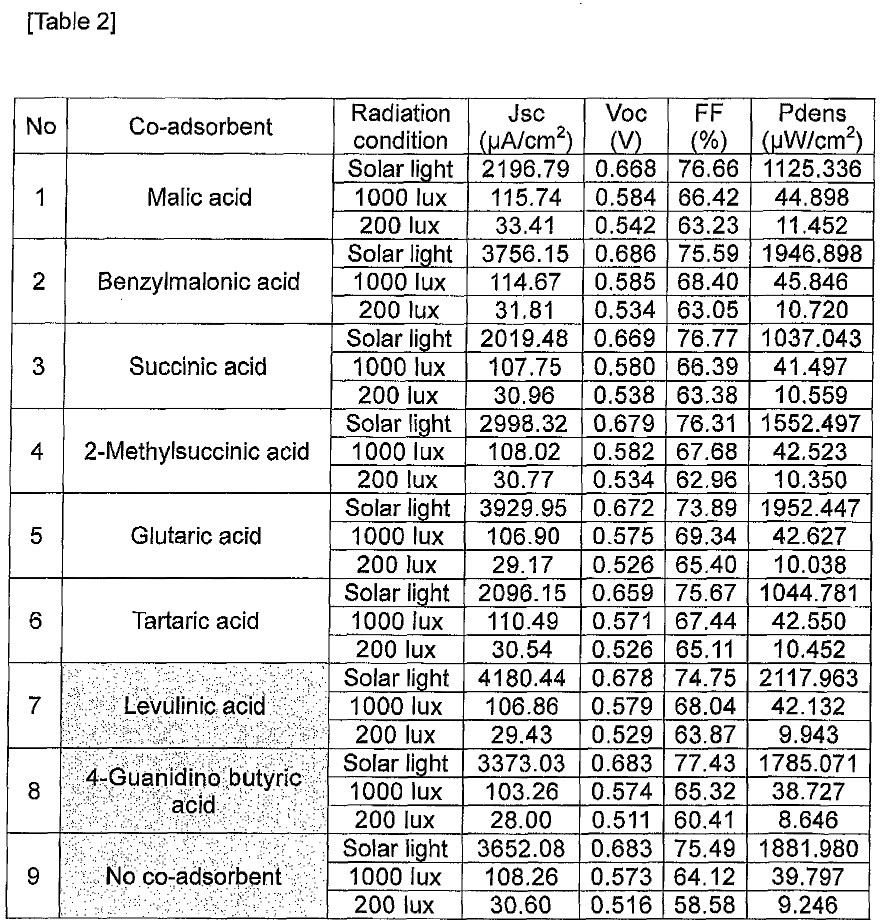

From the above results, even in a case of using the co-adsorbent having a high power generation efficiency under the high illumination circumstance of the solar light and 1000 lux, the obtained DSC did not necessarily show a high conversion efficiency, and the like, under the low illumination circumstance of 200 lux. Particularly, under the low illumination circumstance of 200 lux, in a case of using the co-adsorbents of Nos. 1 to 6, i.e. malic acid, benzylmalonic acid, succinic acid, 2-methylsuccinic acid, glutaric acid, and tartaric acid which have two carboxyl groups at the both ends of a hydrocarbon group having 1 or more and 3 or less carbon atoms, the respective properties of J_{sc}, V_{oc}, FF and P_{dens} were high. To the contrary, in a case of using the levulinic acid of No. 7 and 4-guanidino butyric acid of No. 8 which have only one carboxyl group, or in a case of not using co-adsorbent of No. 9, the properties of J_{sc}, V_{oc}, FF and P_{dens} were low as the whole, and particularly the P_{dens} were all 10 µW/cm² or less.

### INDUSTRIAL APPLICABILITY

The present invention contributes to the improvement of power generation efficiency under a low illumination circumstance such as an indoor living room to extend the use of the DSC in the dye-sensitized solar cell (DSC) having an excellent design property.

### REFERENCE SIGNS LIST

- C: Dye
- A: Co-adsorbent
- Ox: Oxide, Oxide layer
- L: Electrolyte
- E_{c}: Electrode
- E_{Pt}: Counter electrode
- S: Substrate
- R: Light
- e⁻: Electron

## Claims

1. A dye-sensitized solar cell comprising: an electrode where an oxide layer carrying a photosensitizing dye and a co-adsorbent is provided on a substrate; a counter electrode opposite to the electrode; and an electrolyte between the electrode and the counter electrode, wherein:
the photosensitizing dye is an asymmetric ruthenium complex shown in the following general formula (1) where two thiocyanate groups and two bipyridine ligands which are different from each other are coordinated with a Ru metal; and
the co-adsorbent is an organic compound shown in the following general formula (2) where two carboxyl groups are coordinated with a hydrocarbon group (Y) which has a linear saturated hydrocarbon having 1 or more and 3 or less carbon atoms as a main chain. 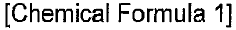 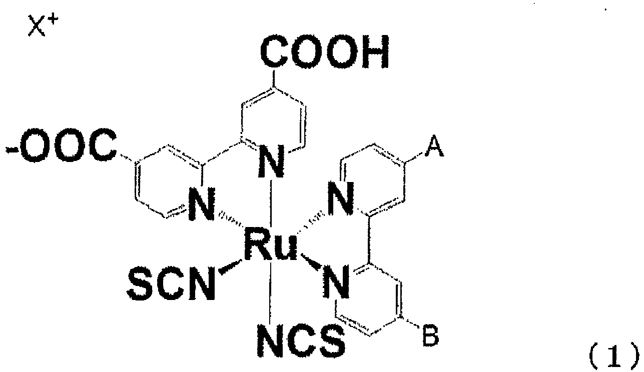
(wherein A and B are a linear hydrocarbon group (having 1 or more and 12 or less carbon atoms), or a substituent which has one or more heterocycles with sulfur or oxygen as a hetero atom. x is a monovalent cation which can be dissociated in a solvent.) 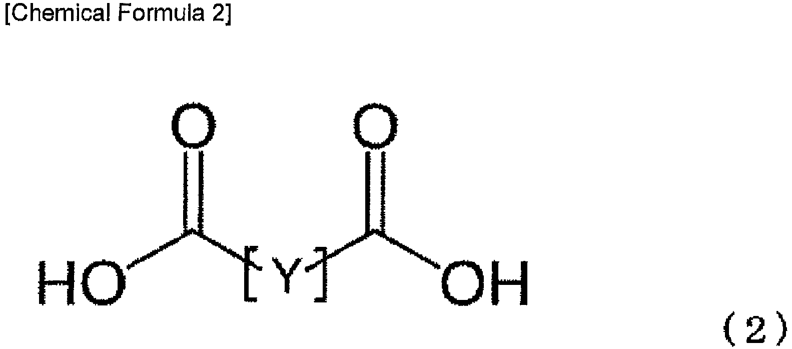 (wherein Y is a hydrocarbon group which is a linear saturated hydrocarbon having 1 or more and 3 or less carbon atoms as a main chain.)

2. The dye-sensitized solar cell according to claim 1, wherein Y is a hydrocarbon group which is a saturated hydrocarbon having 1 or more and 2 or less carbon atoms as a main chain.

3. The dye-sensitized solar cell according to claim 1 or 2, wherein Y is a hydrocarbon group where the main chain composed of the saturated hydrocarbon has a substituent selected from hydrogen atom, a chain or cyclic hydrocarbon group or hydroxyl group as a side chain.

4. The dye-sensitized solar cell according to any of claims 1 to 3, wherein the co-adsorbent is any one or more of malic acid, 2-methylmalonic acid, benzylmalonic acid, succinic acid, 2-methylsuccinic acid, glutaric acid, and tartaric acid.

5. The dye-sensitized solar cell according to any of claims 1 to 4, wherein A and B are the same substituent.

6. The dye-sensitized solar cell according to any of claims 1 to 5, wherein A and/or B have/has a heterocycle of 5-membered ring or 6-membered ring.

7. The dye-sensitized solar cell according to any of claims 1 to 6, wherein A and/or B have/has a biothiophene group.

8. The dye-sensitized solar cell according to any of claims 1 to 7, wherein A and/or B are/is a saturated hydrocarbon group having 1 or more and 12 or less carbon atoms or a biothiophene group having a thioether group which has a saturated hydrocarbon group having 1 or more and 9 or less carbon atoms as a side chain.

9. The dye-sensitized solar cell according to any of claims 1 to 8, wherein X is hydrogen ion, an alkali metal ion or an alkyl ammonium ion ((CₙH₂ₙ₋₁)₄N⁺, n being 1 or more and 6 or less).

10. The dye-sensitized solar cell according to any of claims 1 to 9, wherein a molar ratio of the photosensitizing dye and the co-adsorbent (photosensitizing dye / co-adsorbent) is 0.1 or more and 5 or less.
